(19) Europäisches Patentamt
European Patent Office
Office européen des brevets

(11) Publication number: 0 273 523 B1

(12) # EUROPEAN PATENT SPECIFICATION

(45) Date of publication of the patent specification: **21.03.90**

(51) Int. Cl.⁴: **C07C 303/44**, C07C 309/04

(21) Application number: **87202569.7**

(22) Date of filing: **18.12.87**

(54) **Process for the separation of sulphuric acid from aqueous mixtures thereof with paraffinsulphonic acids.**

(30) Priority: **23.12.86 IT 2281586**

(43) Date of publication of application: **06.07.88 Bulletin 88/27**

(45) Publication of the grant of the patent: **21.03.90 Bulletin 90/12**

(84) Designated Contracting States: **AT BE CH DE ES FR GB GR LI LU NL SE**

(56) References cited:
**EP-A- 0 037 883**
**EP-A- 0 054 851**
**EP-A- 0 158 235**
**GB-A- 1 194 699**

(73) Proprietor: **ENIRICERCHE S.p.A., Corso Venezia 16, I-20121 Milan(IT)**
Proprietor: **ENICHEM AUGUSTA S.p.A., Via Ruggero Settimo 55, I-90139 Palermo(IT)**

(72) Inventor: **Faggian, Lucio, Via Kennedy 32, I-20097 San Donato Milanese Milan(IT)**
Inventor: **Borgarello, Enrico, Corso Grosseto 274, I-10151 Torino(IT)**
Inventor: **Franco, Cosimo, Via Bari 3, I-89044 Locri Reggio Calabria(IT)**
Inventor: **Carrillo, Gerardo, Via Morandi 26, I-20097 San Donato Milanese Milan(IT)**

(74) Representative: **Roggero, Sergio et al, Ing. Barzanò & Zanardo Milano S.p.A. Via Borgonuovo 10, I-20121 Milano(IT)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

The present invention relates to a process for the separation of sulphuric acid from aqueous mixtures thereof with paraffin-sulphonic acids.

The mixtures from which sulphuric acid must be separated according to the present invention are those which derive from the sulphoxidation of ($C_{12}$-$C_{18}$)-n-paraffins with $SO_2$ and $O_2$ in the presence of water and of U.V. light at a temperature comprised within the range of from 25 to 50°C, after the removal of n-paraffins, which separate spontaneously, and of the excess of $SO_2$, and after undergoing one of the following treatments:

a) dehydration, by known systems, of the residual mixture, at least until said mixture becomes cloudy (due to the formation of a biphasic system), extraction of not-sulphoxidated paraffins from the dehydrated, cloudy mixture, or from the supernatant phase of the biphasic system, with supercritical $CO_2$ at a temperature comprised within the range of from 32°C to 80°C, under a pressure comprised within the range of from 75 to 350 bars, and with a $CO_2$/paraffin-sulphonic acids weight ratio of from 1/1 to 50/1.

b) addition of $H_2SO_4$ to the residual mixture, at least until said mixture becomes cloudy (due to the formation of a biphasic system), extraction, with supercritical $CO_2$, from the cloudy mixture or from the supernatant phase of the biphasic system, of the residual paraffins under such conditions as shown under (a);

c) addition to the residual mixture of an aliphatic alcohol containing a number of carbon atoms lower than, or equal to, 4, preferably isopropanol, until a biphasic mixture is formed, extraction from said biphasic mixture of the residual paraffins with supercritical $CO_2$ under such conditions as reported under (a).

The mixture of paraffin-sulphonic acids free, or substantially free, from paraffins, obtained by means of the above disclosed treatments from (a) to (c) (the "refined mixture") still contains, besides the paraffin-sulphonic acids, a considerable amount of $H_2SO_4$.

The purpose of the process of the present invention is to remove said excess of sulphuric acid.

The compositions of the refined mixtures of paraffin-sulphonic acids obtained by means of the methods herein previously mentioned from (a) to (c) are the following:

| | | |
|---|---|---|
| 1) | ($C_{12}$–$C_{18}$)-Paraffin-sulphonic acids: | from 3 to 83% by weight |
| 2) | $H_2O$: | from 79 to 8.5% by weight |
| 3) | $H_2SO_4$: | from 18 to 8.5% by weight |
| 4) | ($C_{12}$–$C_{18}$)-Paraffins: | less than 1% relatively to ($C_{12}$–$C_{18}$)-paraffin-sulphonic acids |

Obviously, the starting mixture, even if it is obtained by the present Applicants in the above 3 ways, can be obtained in other ways too, so that the present invention should not be considered as being limited to the way in which the starting mixture with the above reported composition is obtained, in as much as the process of the invention can be applied to any mixtures, in whatever way they are obtained, having the above compositions.

The process according to the present invention comprises mixing the refined mixture, having the above-said compositions, at a temperature comprised within the range of from 10 to 80°C, preferably of from 20 to 50°C, with one or more halogenated solvent(s) selected from those defined by the general formulae (1), (2), (3):

(1) 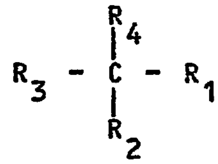

(2) 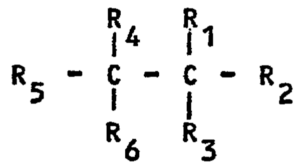

(3) 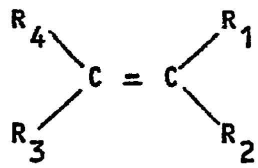

wherein at least one of $R_1$, $R_2$, $R_3$, $R_4$, $R_5$, $R_6$ is a halogen, the other R radicals being H, the halogen being fluorine and/or chlorine and/or bromine and/or iodine, with a phase constituted by $H_2SO_4$ and water being separated from a phase containing the residual refined mixture, optionally mixing the residual refined mixture with $H_2SO_4$ (ranging from an aqueous $H_2SO_4$ having a minimum concentration of 70% by weight of $H_2SO_4$ to concentrated $H_2SO_4$, or oleum, or even $SO_3$), at a temperature comprised within the range of from 10°C to 80°C, preferably of from 20°C to 50°C, in such way a second phase, which is constituted by $H_2SO_4$ and $H_2O$, being separated from the residual refined mixture, submitting the residual refined mixture to a treatment of separation of the halogenated solvent and/or halogenated solvents, used, in particular by distillation at a temperature lower than 100°C, preferably lower than 50-60°C, still more preferably at least partially under vacuum.

The amount of halogenated solvent(s) added depends also on the type of the solvents, and on the composition of the refined mixture, and is anyway such as to make it possible an as large as possible amount of $H_2SO_4$ to be separated.

The optionally added amount of $H_2SO_4$ is, in case of $H_2SO_4$ at 96% of concentration, comprised within the range of from 0% to 200% by weight, preferably of from 50% to 150% by weight, relatively to the weight of the paraffin-sulphonic acids comprised in the mixture.

According to a form of practical embodiment of the process according to the present invention, the halogenated solvent(s) and sulphuric acid can be simultaneously mixed with the refined mixture in a single processing step.

Among the halogenated solvents, preferred are methylene chloride, chloroform, carbon tetrachloride and dichloroethane.

Residual $H_2SO_4$, if any, or, partially, $H_2SO_4$ present before the application of the process according to the present invention, can be removed by being converted into an insoluble product by means of the addition of carbonates, hydroxides or oxides of alkaline-earth metals, in particular, by means of the addition of calcium carbonate, calcium hydroxide or calcium oxide.

Some examples are now given for the purpose of better illustrating the invention, it being understood that the same invention has not to be considered as being limited to them or by them.

<u>Example 1</u>

To a glass dropping funnel, tightly sealable, 20.47 g is charged of a mixture having the following composition:

| | |
|---|---|
| Paraffin-sulphonic acids: | 59.95% by weight |
| ($C_{12}$–$C_{18}$)-n-paraffins: | 0.22% by weight |
| Water: | 28.72% by weight |
| Sulphuric acid: | 11.11% by weight |

which had been obtained by means of the extraction with supercritical $CO_2$ of the n-paraffins contained in the upper phase of a raw mixture (from which the decantable n-paraffins and $SO_2$ had been removed) of paraffin-sulphonic acids, obtained by means of the sulphoxidation of ($C_{12}$-$C_{18}$)-n-paraffins, having the following composition:

| | |
|---|---|
| Paraffin-sulphonic acids: | 24.74% by weight |
| $(C_{12}-C_{18})$-n-paraffins: | 26.46% by weight |
| Water: | 40.94% by weight |
| Sulphuric acid: | 7.86% by weight |

after the addition of 20% by weight of $H_2SO_4$ at 96% by weight, referred to the weight of said raw mixture, as reported under (c) (European Patent Application No.87201344.6)

| | | |
|---|---|---|
| $CO_2$/paraffinsulphonic acids ratio | = | 15.5 |
| Extraction pressure | = | 150 bar |
| Extraction temperature | = | 45°C |
| Extraction time | = | 1 hour |

To the dropping funnel, 30.69 g of $CH_2Cl_2$ is then added, the dropping funnel is thoroughly shaken for a few minutes, and the phases are allowed to separate. After 3 hours of standing at 22°C, the two separated phases are taken away from each other, and are analysed.

The lower phase is constituted by water (55.44% by weight), sulphuric acid (44.02% by weight) and traces of $CH_2Cl_2$.

In the upper phase, all charged paraffin-sulphonic acids are present, together with minor amounts of water, sulphuric acid, n-paraffins, besides $CH_2Cl_2$. In particular, the content of $H_2SO_4$ referred to the present paraffin-sulphonic acids, has decreased from 18.5% by weight (before the treatment with $CH_2Cl_2$) to 8.36% by weight. Also the water content, still referred to paraffin-sulphonic acids, has decreased from 47.9% by weight to 31.7% by weight.

Aliquots of the upper phase, obtained by means of the treatment with $CH_2Cl_2$, as previously disclosed, having the composition:

| | |
|---|---|
| Paraffin-sulphonic acids: | 25.92% by weight |
| $(C_{12}-C_{18})$-n-paraffins: | 0.094% by weight |
| Water: | 8.21% by weight |
| Sulphuric acid: | 2.168% by weight |
| $CH_2Cl_2$ | the balance to 100 |

are extracted, inside a dropping funnel, with different amounts of $H_2SO_4$ at 96% by weight. After the phase separation, at 22°C, the two phases formed are taken away from each other, and analysed.

The obtained values are reported in Table 1.

Table 1

| Test No. | $CH_2Cl_2$ Phase Charge, g | Added $H_2SO_4$ at 96%, g | Added $H_2SO_4$/paraffin-sulphonic acids % ratio, by weight | Analysis of the upper phase: Paraffin-sulphonic acids, % by weight | Analysis of the upper phase: $H_2O$, % by weight | Analysis of the upper phase: $H_2SO_4$, % by weight | Analysis of the lower phase: Paraffin-sulphonic acids, % by weight | Analysis of the lower phase: $H_2O$, % by weight | Analysis of the lower phase: $H_2SO_4$, % by weight | $H_2SO_4$/paraffin-sulphonic acids ratio in the upper phase, % by weight | $H_2O$/paraffin-sulphonic acids ratio in the upper phase, % by weight |
|---|---|---|---|---|---|---|---|---|---|---|---|
| 1.1 | 10.2143 | 1.0944 | 41.33 | 28.64 | 2.74 | 1.364 | 0 | 35.28 | 63.64 | 4.76 | 9.57 |
| 1.2 | 10.3137 | 1.5468 | 57.87 | 28.51 | 1.94 | 1.268 | 0 | 30.37 | 68.69 | 4.45 | 6.80 |
| 1.3 | 10.6545 | 2.0810 | 75.3 | 29.91 | 1.46 | 1.292 | 0 | 27.50 | 71.08 | 4.14 | 4.88 |

Example 2

To a glass dropping funnel, tightly sealable, 217.8 g is charged of the same mixture as used in Example 1.

652.9 g of $CH_2Cl_2$ is then added. The dropping funnel is thoroughly shaken and the phases are allowed to separate. After standing 24 hours at 22°C, the two separated phases are taken away from each other, and are analysed.

The lower phase is constituted by water (50.73% by weight), sulphuric acid (44.03% by weight) and a small amount of $CH_2Cl_2$.

In the upper phase, all charged paraffin-sulphonic acids are present, together with minor amounts of water, sulphuric acid, n-paraffins, besides $CH_2Cl_2$. In particular, the content of sulphuric acid, referred to the present paraffin-sulphonic acids, has decreased from 18.5% by weight (before the treatment with $CH_2Cl_2$) to 7.49% by weight. Also the water content, still referred to the present paraffin-sulphonic acids, has decreased from 47.9% by weight to 30% by weight.

Aliquots of the upper phase, obtained by means of the treatment with $CH_2Cl_2$, as previously disclosed, having the composition:

| | |
|---|---|
| Paraffin-sulphonic acids: | 16.535% by weight |
| ($C_{12}$–$C_{18}$)-n-paraffins: | 0.060% by weight |
| Water: | 4.96% by weight |
| Sulphuric acid: | 1.238% by weight |
| $CH_2Cl_2$ | the balance to 100 |

are extracted, inside a dropping funnel, with different amounts of $H_2SO_4$ at 96% by weight. After the phase separation, at 22°C, the two phases formed are separated from each other, and analysed.

The obtained values are reported in Table 2.

Example 3

To a dropping funnel, tightly sealable, 22.45 g is charged of the same mixture as used in Example 1.

224.35 g of $CH_2Cl_2$ is added. The dropping funnel is thoroughly shaken and the phases are allowed to separate. After standing 24 hours at 22°C, the two separated phases are taken away from each other, and are analysed.

The lower phase is constituted by water (53.51% by weight), sulphuric acid (42.48% by weight) and minor amounts of $CH_2Cl_2$.

In the upper phase, all charged paraffin-sulphonic acids are present, together with minor amounts of water, sulphuric acid, n-paraffins, besides $CH_2Cl_2$. In particular, the content of sulphuric acid, referred to the present paraffin-sulphonic acids, has decreased from 18.5% by weight (before the treatment with $CH_2Cl_2$) to 6.36% by weight. Also the water content, still referred to the present paraffin-sulphonic acids, has decreased from 47.9% by weight to 33.0% by weight.

Aliquots of the upper phase, obtained by means of the treatment with $CH_2Cl_2$, having the composition:

| | |
|---|---|
| Paraffin-sulphonic acids: | 5.584% by weight |
| ($C_{12}$–$C_{18}$)-n-paraffins: | 0.020% by weight |
| Water: | 1.840% by weight |
| Sulphuric acid: | 0.355% by weight |
| $CH_2Cl_2$ | the balance to 100 |

are extracted, inside a dropping funnel, with different amounts of $H_2SO_4$ at 96% by weight. After the phase separation, at 22°C, the two phases formed are separated from each other, and analysed.

The obtained values are reported in Table 3.

Table 2

| Test No. | $CH_2Cl_2$ Phase Charge, g | Added $H_2SO_4$ at 96%, g | Added $H_2SO_4$/paraffin-sulphonic acids % ratio, by weight | Analysis of the upper phase: Paraffin-sulphonic acids, % by weight | Analysis of the upper phase: $H_2O$, % by weight | Analysis of the upper phase: $H_2SO_4$, % by weight | Analysis of the lower phase: Paraffin-sulphonic acids, % by weight | Analysis of the lower phase: $H_2O$, % by weight | Analysis of the lower phase: $H_2SO_4$, % by weight | $H_2SO_4$/paraffin-sulphonic acids ratio in the upper phase, % by weight | $H_2O$/paraffin-sulphonic acids ratio in the upper phase, % by weight |
|---|---|---|---|---|---|---|---|---|---|---|---|
| 2.1 | 23.8090 | 3.5494 | 90.2 | 16.60 | 0.499 | 0.496 | 0 | 24.41 | 72.84 | 2.99 | 3.01 |
| 2.2 | 24.1925 | 5.9650 | 149.1 | 16.49 | 0.176 | 0.594 | 0 | 19.42 | 79.73 | 3.60 | 1.07 |
| 2.3 | 24.1608 | 8.3762 | 209.7 | 16.52 | 0.118 | 0.914 | 0 | 15.55 | 83.12 | 5.53 | 0.71 |
| 4.1 | 17.7698 | 1.2404 | 45.15 | 16.28 | 1.306 | 0.586 | 0 | 33.43 | 65.66 | 3.60 | 8.02 |
| 4.2 | 19.0743 | 2.0422 | 69.25 | 16.46 | 0.801 | 0.511 | 0 | 28.06 | 71.515 | 3.10 | 4.87 |
| 4.3 | 17.9379 | 2.5976 | 93.67 | 16.40 | 0.498 | 0.502 | 0 | 25.02 | 74.18 | 3.06 | 3.04 |

Table 3

| Test No. | $CH_2Cl_2$ Phase Charge, g | Added $H_2SO_4$ at 96%, g | Added $H_2SO_4$/paraffin-sulphonic acids % ratio, by weight | Analysis of the upper phase | | | Analysis of the lower phase | | | $H_2SO_4$/paraffin-sulphonic acids ratio in the upper phase, % by weight | $H_2O$/paraffin-sulphonic acids ratio in the upper phase, % by weight |
|---|---|---|---|---|---|---|---|---|---|---|---|
| | | | | Paraffin-sulphonic acids, % by weight | $H_2O$, % by weight | $H_2SO_4$, % by weight | Paraffin-sulphonic acids, % by weight | $H_2O$, % by weight | $H_2SO_4$, % by weight | | |
| 3.1 | 38.6158 | 1.9658 | 91.2 | 5.809 | 0.139 | 0.091 | 0 | 25.14 | 71.30 | 1.57 | 2.39 |
| 3.2 | 38.1813 | 3.7011 | 173.6 | 5.806 | 0.0317 | 0.123 | 0 | 17.84 | 79.05 | 2.12 | 0.55 |
| 3.3 | 38.2163 | 1.3300 | 62.32 | 5.720 | 0.260 | 0.106 | 0 | 29.15 | 68.00 | 1.85 | 4.55 |
| 3.4 | 38.0728 | 0.7079 | 33.30 | 5.640 | 0.491 | 0.152 | 0 | 36.63 | 61.26 | 2.70 | 8.71 |
| 3.5 | 27.9606 | 1.9132 | 122.6 | 5.804 | 0.080 | 0.142 | 0 | 21.35 | 76.47 | 2.45 | 1.38 |

Example 4

To a dropping funnel, tightly sealable, withstanding moderate pressures, 16.1 g is charged of the same mixture as used in Example 1.

48.6 g of $CH_2Cl_2$ is added. The dropping funnel is thoroughly shaken and is placed inside an oven maintained at the controlled temperature of 40°C. After 30 minutes, the dropping funnel is thoroughly shaken once more inside the oven. The phases are allowed to separate. After standing 16 hours at 40°C, the lower phase is removed, with the dropping funnel being kept inside the oven. The funnel containing the upper phase is removed from the oven, and is cooled to room temperature. The two phases are analysed.

The lower phase is constituted by water (53.47% by weight), sulphuric acid (42.90% by weight) and minor amounts of $CH_2Cl_2$.

In the upper phase, all charged paraffin-sulphonic acids are present, together with minor amounts of water, sulphuric acid, n-paraffins, besides $CH_2Cl_2$. In particular, the content of sulphuric acid, referred to the present paraffin-sulphonic acids, has decreased from 18.5% by weight (before the treatment with $CH_2Cl_2$) to 5.58% by weight. Also the water content, still referred to the present paraffin-sulphonic acids, has decreased from 47.9% by weight to 27.22% by weight.

Aliquots of the upper phase, obtained by means of the treatment with $CH_2Cl_2$, having the composition:

| | |
|---|---|
| Paraffin-sulphonic acids: | 15.98% by weight |
| ($C_{12}$–$C_{18}$)-n-paraffins: | 0.058% by weight |
| Water: | 4.35% by weight |
| Sulphuric acid: | 0.892% by weight |
| $CH_2Cl_2$ | the balance to 100 |

are extracted, inside a tightly sealable dropping funnel withstanding moderate pressures, with different amounts of $H_2SO_4$ at 96% by weight, by operating at 40°C inside an oven.

After the phase separation, at 40°C, the lower phase is removed, by operating inside the oven.

The funnel is then removed from the oven, and is made cool to room temperature. The two phases are analysed.

The obtained values are reported in Table 4.

Table 4

| Test No. | $CH_2Cl_2$ Phase Charge, g | Added $H_2SO_4$ at 96%, g | Added $H_2SO_4$/paraffin-sulphonic acids % ratio, by weight | Analysis of the upper phase: Paraffin-sulphonic acids, % by weight | Analysis of the upper phase: $H_2O$, % by weight | Analysis of the upper phase: $H_2SO_4$, % by weight | Analysis of the lower phase: Paraffin-sulphonic acids, % by weight | Analysis of the lower phase: $H_2O$, % by weight | Analysis of the lower phase: $H_2SO_4$, % by weight | $H_2SO_4$/paraffin-sulphonic acids ratio in the upper phase, % by weight | $H_2O$/paraffin-sulphonic acids ratio in the upper phase, % by weight |
|---|---|---|---|---|---|---|---|---|---|---|---|
| 6.1 | 11.4404 | 1.0814 | 59.2 | 17.005 | 0.860 | 0.497 | 0 | 29.71 | 69.57 | 2.92 | 5.06 |
| 6.2 | 12.7039 | 2.0280 | 99.9 | 17.090 | 0.355 | 0.447 | 0 | 24.60 | 75.7 | 2.62 | 2.08 |
| 6.3 | 10.9668 | 2.2819 | 130.2 | 16.890 | 0.243 | 0.570 | 0 | 21.32 | 78.88 | 3.37 | 1.44 |

Example 5

To a dropping funnel, tightly sealable, withstanding moderate pressures, 23.0 g is charged of the same mixture of paraffin-sulphonic acids as used in Example 1.

142.6 g of $CH_2Cl_2$ is added, and the dropping funnel is thoroughly shaken. The dropping funnel is then placed inside an oven maintained at the controlled temperature of 40°C. After 1 hour, the dropping funnel is thoroughly shaken once more, by operating inside the oven. The phases are allowed to separate. After standing 26 hours at 40°C, the lower phase is removed from the interior of the oven. The funnel containing the upper phase is removed from the oven, and is allowed to cool down to room temperature. The two phases are analysed.

The lower phase (4.4391 g) is constituted by water (56.66% by weight), sulphuric acid (43.08% by weight) and minor amounts of $CH_2Cl_2$.

In the upper phase (160.8172 g), all of the charged paraffin-sulphonic acids are present, together with minor amounts of water, sulphuric acid, n-paraffins, besides $CH_2Cl_2$. In particular, the content of sulphuric acid, referred to the present paraffin-sulphonic acids, has decreased from 18.5% by weight (before the treatment with $CH_2Cl_2$) to 4.84% by weight. Also the water content, still referred to the present paraffin-sulphonic acids, has decreased from 47.9% by weight to 28.11% by weight.

To 148.1 g of the upper phase, inside a dropping funnel resistant to moderate pressures, 11.5 g of $H_2SO_4$ at 96% by weight is charged.

The dropping funnel is thoroughly shaken, and is placed inside an oven maintained at the controlled temperature of 40°C. After 1 hour, the dropping funnel is thoroughly shaken once more and is then left standing 4 hours at 40°C.

By operating inside the oven, the lower phase (15.2294 g) is removed.

The funnel containing the upper phase (143.9 g) is removed from the oven, and is allowed to cool down to room temperature. The two phases are analysed.

The lower phase contains water (25.5% by weight), sulphuric acid (73.6% by weight), together with minor amounts of $CH_2Cl_2$ (the balance to 100%), and does not contain paraffin-sulphonic acids.

The upper phase has the following composition:

| | |
|---|---|
| Paraffin-sulphonic acids: | 9.07% by weight |
| ($C_{12}$–$C_{18}$)-n-paraffins: | 0.033% by weight |
| Water: | 0.230% by weight |
| Sulphuric acid: | 0.198% by weight |
| $CH_2Cl_2$ | the balance to 100 |

The $H_2SO_4$/paraffin-sulphonic acids ratio results to be 2.18%; the water/paraffin-sulphonic acids ratio results to be 2.54%. 134 g of this upper phase is fed, in continuous mode, to a rotary evaporator operating under a slight vacuum (120-130 $mm_{Hg}$), and with the temperature of the heating bath being of 50-55°C. When all of the product has been fed, and approximately the total amount of $CH_2Cl_2$ has evaporated, the vacuum is increased up to 700 $mm_{Hg}$. The whole process step lasts about 1 hour.

The residual product remaining inside the kettle of the rotary evaporator is a thin liquid essentially constituted by paraffin-sulphonic acids, with 1.585% by weight of $H_2O$, 2.182% by weight of $H_2SO_4$ and 0.360% by weight of ($C_{12}$-$C_{18}$)-n-paraffins.

The distribution of monosulphonic, disulphonic and trisulphonic acids found in the concentrated end product results to be the same as found in the raw mixture of paraffin-sulphonic acids downstream the sulphoxidation reactor.

Example 6

By operating at 22°C, and using the same mixture of paraffin-sulphonic acids as used in Example 1, tests of purification of the paraffin-sulphonic acids from sulphuric acids, using different solvents, were carried out.

The results obtained are shown in Table 5.

It can be observed how only the halogenated solvents supply interesting results.

Table 5

| Test No. | Type of solvent | Grams of solvent | Grams of paraffin-sulphonic mixture | Separated organic phase, g | Separated aqueous phase, g | Ratio, by weight, of $H_2SO_4$/paraffin-sulphonic acids in organic phase, % | Ratio, by weight, of $H_2O$/paraffin-sulphonic acids in organic phase, % | REMARKS |
|---|---|---|---|---|---|---|---|---|
| 1 | $CHCl_3$ | 3.4815 | 3.2700 | 6.0640 | 0.5247 | 4.12 | 32.2 | |
| 2 | $CCl_4$ | 2.5884 | 3.4649 | 5.7156 | 0.3264 | 7.79 | 38.3 | |
| 3 | $CH_2Cl-CH_2Cl$ | 3.0531 | 3.4600 | 5.1625 | 0.4214 | 10.2 | 35.4 | Also present in the intermediate phase of $CH_2Cl-CH_2Cl$ |
| 4 | $CCl_2=CH_2$ | 2.6188 | 3.4609 | 5.6313 | 0.4488 | 10.99 | 33.7 | |
| 5 | $(C_2H_5)_2O$ | 1.7001 | 3.3640 | 4.9189 | 0.0810 | 16.26 | 40.6 | |
| 6 | $CH_3COOC_2H_5$ | 2.3794 | 3.4308 | | | | | Single phase |
| 7 | Petroleum ether, 40-70$^{0}$C | 1.9217 | 3.3102 | | | | | Single phase |

Example 7

To a tightly sealable test tube, 7.4428 g is charged of the same mixture of paraffin-sulphonic acids as used in Example 1.

14.7382 g of $CHCl_3$ is added. The test tube is thoroughly shaken, and is then left standing at 23°C for 6.5 hours, and is then centrifuged, to favour the phase separation. The two phases are taken away from each other, and are analysed.

The lower phase (1.3330 g) is essentially constituted by water (57.5% by weight) and sulphuric acid (42.46% by weight).

In the upper phase (20.7284 g), all of the charged paraffin-sulphonic acids are present, together with minor amounts of water, sulphuric acid, n-paraffins, besides $CHCl_3$. In particular, the content of sulphuric acid, referred to the present paraffin-sulphonic acids, has decreased from 18.5% by weight (before the treatment with $CHCl_3$) to 5.74% by weight.

Also the water content, still referred to the present paraffin-sulphonic acids, has decreased from 47.9% by weight to 29.35% by weight.

To 10.5305 g of the beforehand separated upper phase, 2.0448 g of $H_2SO_4$ at 96% by weight is charged.

The test tube is thoroughly shaken, and is left standing at 23°C. After 7 hours, the two phases are taken away from each other, and are analysed.

The lower phase contains water (25.08% by weight), sulphuric acid (73.44% by weight), together with a small amount of $CHCl_3$, and does not contain paraffin-sulphonic acids.

The upper phase has the following composition:

| | |
|---|---|
| Paraffin-sulphonic acids: | 22.75% by weight |
| ($C_{12}$–$C_{18}$)-n-paraffins: | 0.082% by weight |
| Water: | 0.80% by weight |
| Sulphuric acid: | 0.686% by weight |
| $CHCl_3$ | the balance to 100 |

The $H_2SO_4$/paraffin-sulphonic acids ratio result to be 3.02%; the water/paraffin-sulphonic acids ratio results to be 3.52%.

## Claims

1. Process for the separation of sulphuric acid from aqueous mixtures thereof with ($C_{12}$-$C_{18}$)-paraffin-sulphonic acids, wherein said mixtures contain an amount of ($C_{12}$-$C_{18}$)-paraffin-sulphonic acids comprised within the range of from 3 to 83% by weight; an amount of water comprised within the range of from 8.5 to 79% by weight; an amount of $H_2SO_4$ comprised within the range of from 8.5 to 18% by weight; and an amount of ($C_{12}$-$C_{18}$)-n-paraffins smaller than 1% by weight, relatively to the weight of ($C_{12}$-$C_{18}$)-paraffin-sulphonic acids, characterized in that said mixtures are mixed, at a temperature comprised within the range of from 10 to 80°C, with one or more halogenated solvent(s) selected from those defined by the general formulae (1), (2), (3):

(1) 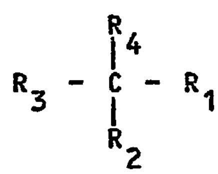

(2) 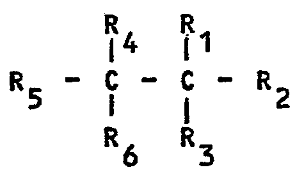

(3) 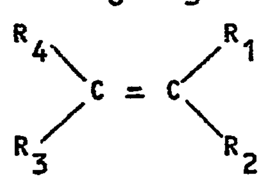

wherein at least one of $R_1$, $R_2$, $R_3$, $R_4$, $R_5$ and $R_6$ is a halogen, the other R radicals being H, with a phase constituted by $H_2SO_4$ and $H_2O$ being separated from a phase containing the residual mixture, the residual mixture is optionally mixed with $H_2SO_4$ in such way a second phase, which is constituted by $H_2SO_4$ and $H_2O$, being separated from the remainder of the mixture, the remainder of the mixture being submitted to the separation of the halogenated solvent(s) used, by distillation at a temperature lower than 100°C.

2. Process according to claim 1, characterized in that the halogenated solvent is selected from chloroform, methylene chloride, carbon tetrachloride and dichloroethane.

3. Process according to claim 1, characterized in that the $H_2SO_4$ used in the optional mixing is mixed at a temperature comprised within the range of from 10 to 80°C.

4. Process according to claim 3, characterized in that $H_2SO_4$ is aqueous $H_2SO_4$ having a minimum concentration of 70% of $H_2SO_4$.

5. Process according to claim 3, characterized in that $H_2SO_4$ is concentrated $H_2SO_4$, in particular $H_2SO_4$ at 96%.

6. Process according to claim 3, characterized in that $H_2SO_4$ is oleum.

7. Process according to claim 3, characterized in that $H_2SO_4$ is used in the form of $SO_3$.

8. Process according to claims 1 and 3, wherein the temperature is comprised within the range of from 20 to 50°C.

9. Process according to claim 1, characterized in that the removal of the halogenated solvent(s) by distillation is carried out at a temperature lower than 50–60°C.

10. Process according to claims 1 and 9, characterized in that the removal of the halogenated solvent(s) is carried out under vacuum.

11. Process according to claim 1, characterized in that the halogenated solvent(s) and sulphuric acid are simultaneously admixed to the mixture of paraffin-sulphonic acids in one single step.

12. Process according to claim 1, characterized in that $H_2SO_4$ still present inside the end mixture of paraffin-sulphonic acids is removed by insolubilization, carried out by means of the addition of carbonates, hydroxides or oxides of alkaline-earth metals.

## Revendications

1. Procédé de séparation de l'acide sulfurique à partir de mélanges aqueux de celui-ci avec des acides paraffine-sulfoniques en $C_{12}$–$C_{18}$, dans lesquels lesdits mélanges contiennent une proportion d'acides paraffine-sulfoniques en $C_{12}$–$C_{18}$ comprise entre 3 et 83% en poids, une proportion d'eau comprise entre 8,5 et 79% en poids, une proportion de $H_2SO_4$ comprise entre 8,5 et 18% en poids, et une proportion de n-paraffines en $C_{12}$–$C_{18}$ inférieure à 1% en poids, rapportée au poids des acides paraffine-sulfoniques en $C_{12}$-$C_{18}$, caractérisé en ce que lesdits mélanges sont mélangés, à une température comprise entre 10 et 80°C, avec un ou plusieurs solvants halogénés choisis parmi ceux définis par les formules générales (1), (2) et (3):

(1) 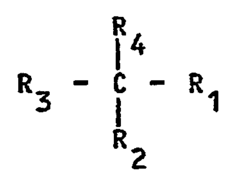

(2) 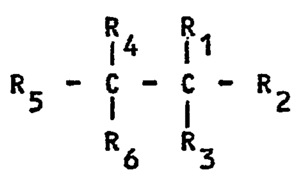

(3) 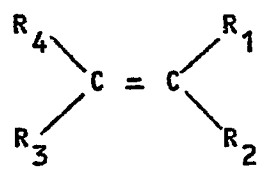

dans lesquelles au moins l'un des $R_1$, $R_2$, $R_3$, $R_4$, $R_5$ et $R_6$ est un atome d'halogène, les autres radicaux R représentant H, une phase constituée de $H_2SO_4$ et de $H_2O$ est séparée d'une phase contenant le mélange résiduel, ce mélange résiduel est éventuellement mélangé avec du $H_2SO_4$, de telle façon qu'une seconde phase, constituée de $H_2SO_4$ et de $H_2O$, se sépare du reste du mélange, et ce reste du mélange est soumis à la séparation du ou des solvant(s) halogéné(s) utilisé(s), par distillation à une température inférieure à 100°C.

2. Procédé conforme à la revendication 1, caractérisé en ce que le solvant halogéné est choisi parmi le chloroforme, le chlorure de méthylène, le tétrachlorure de carbone et le dichloroéthane.

3. Procédé conforme à la revendication 1, caractérisé en ce que le $H_2SO_4$ utilisé dans le mélange éventuel est mélangé à une température comprise entre 10 et 80°C.

4. Procédé conforme à la revendication 3, caractérisé en ce que le $H_2SO_4$ est un $H_2SO_4$ aqueux présentant une concentration minimale de 70% de $H_2SO_4$.

5. Procédé conforme à la revendication 3, caractérisé en ce que le $H_2SO_4$ est un $H_2SO_4$ concentré, en particulier du $H_2SO_4$ à 96%.

6. Procédé conforme à la revendication 3, caractérisé en ce que le $H_2SO_4$ est un oléum.

7. Procédé conforme à la revendication 3, caractérisé en ce que le $H_2SO_4$ est utilisé sous forme de $SO_3$.

8. Procédé conforme aux revendications 1 et 3, dans lequel la température est comprise entre 20 et 50°C.

9. Procédé conforme à la revendication 1, caractérisé en ce que l'élimination du ou des solvant(s) halogéné(s) par distillation est effectuée à une température inférieure à 50–60°C.

10. Procédé conforme aux revendications 1 et 9, caractérisé en ce que l'élimination du ou des solvant(s) halogéné(s) est effectuée sous vide.

11. Procédé conforme à la revendication 1, caractérisé en ce que le ou les solvant(s) halogéné(s) et l'acide sulfurique sont simultanément ajoutés et mélangés au mélange d'acides paraffine-sulfoniques, en une seule étape.

12. Procédé conforme à la revendication 1, caractérisé en ce que le $H_2SO_4$ encore présent dans le mélange final d'acides paraffine-sulfoniques est éliminé par insolubilisation, effectuée par addition de carbonates, hydroxydes ou oxydes de métaux alcalinoterreux.

## Patentansprüche

1. Verfahren zur Abtrennung von Schwefelsäure aus wässrigen Gemischen hievon mit ($C_{12}$–$C_{18}$)-Paraffinsulfonsäuren, welche Gemische eine Menge von ($C_{12}$–$C_{18}$)-Paraffinsulfonsäuren im Bereich von 3–83 Gew.-%; eine Menge von Wasser im Bereich von 8,5–79 Gew.-%; eine Menge von $H_2SO_4$ im Bereich von 8,5–18 Gew.-%; und eine Menge an ($C_{12}$–$C_{18}$)-n-Paraffinen von unter 1 Gew.-%, bezogen auf das Gewicht der ($C_{12}$–$C_{18}$)-Paraffinsulfonsäuren, enthalten, dadurch gekennzeichnet, daß diese Gemische bei einer Temperatur im Bereich von 10–80°C mit einem oder mit mehreren halogenierten Lösungsmitteln, ausgewählt unter den durch die allgemeinen Formeln (1), (2) und (3) definierten Lösungsmitteln:

(1) 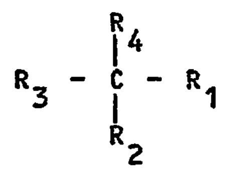

(2) 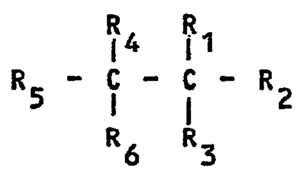

(3) 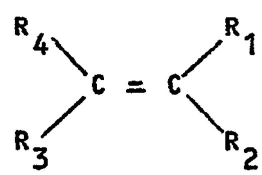

worin wenigstens einer der Reste $R_1$, $R_2$, $R_3$, $R_4$, $R_5$, und $R_6$ ein Halogen bedeutet und die anderen Reste R für Wasserstoff stehen, vermischt werden, eine aus $H_2SO_4$ und $H_2O$ bestehenden Phase von einer das restliche Gemisch enthaltenden Phase abgetrennt wird, das restliche Gemisch gewünschtenfalls mit $H_2SO_4$ vermischt wird, in welcher Weise eine zweite Phase, die aus $H_2SO_4$ und $H_2O$ gebildet ist, vom Rest des Gemisches abgetrennt wird, und das verbleibende Gemisch einer Abtrennung des oder der verwendeten halogenierten Lösungsmittel durch Destillation bei einer unter 100°C liegenden Temperatur unterworfen wird.

2. Verfahren nach Anspruch 1, dadurch gekennzeichnet, daß das halogenierte Lösungsmittel unter Chloroform, Methylenchlorid, Tetrachlorkohlenstoff und Dichlorethan ausgewählt wird.

3. Verfahren nach Anspruch 1, dadurch gekennzeichnet, daß die im fakultativen Mischvorgang verwendete Schwefelsäure bei einer Temperatur im Bereich von 10 bis 80°C vermischt wird.

4. Verfahren nach Anspruch 3, dadurch gekennzeichnet, daß die Schwefelsäure eine wäßrige Schwefelsäure mit einer Mindestkonzentration von 70% $H_2SO_4$ ist.

5. Verfahren nach Anspruch 3, dadurch gekennzeichnet, daß die Schwefelsäure konzentrierte Schwefelsäure ist, inbesondere 96%ige $H_2SO_4$.

6. Verfahren nach Anspruch 3, dadurch gekennzeichnet, daß die Schwefelsäure Oleum ist.

7. Verfahren nach Anspruch 3, dadurch gekennzeichnet, daß die Schwefelsäure in Form von $SO_3$ eingesetzt wird.

8. Verfahren nach den Ansprüchen 1 und 3, worin die Temperatur im Bereich von 20 bis 50°C liegt.

9. Verfahren nach Anspruch 1, dadurch gekennzeichnet, daß die Abtrennung des oder der halogenierten Lösungsmittel durch Destillation bei einer unter 50 bis 60°C liegenden Temperatur ausgeführt wird.

10. Verfahren nach den Ansprüchen 1 und 9, dadurch gekennzeichnet, daß die Abtrennung des oder der halogenierten Lösungsmittel unter Vakuum vorgenommen wird.

11. Verfahren nach Anspruch 1, dadurch gekennzeichnet, daß das halogenierte Lösungsmittel bzw. die halogenierten Lösungsmittel und Schwefelsäure gleichzeitig dem Gemisch der Paraffinsulfonsäuren in einer einzigen Stufe zugemischt werden.

12. Verfahren nach Anspruch 1, dadurch gekennzeichnet, daß die im Endgemisch der Paraffinsulfonsäuren noch vorliegende Schwefelsäure durch Insolubilisierung abgetrennt wird, die durch Zugabe von Karbonaten, Hydroxiden oder Oxiden von Erdalkalimetallen vorgenommen wird.